# EUROPEAN PATENT SPECIFICATION

(11) **EP 3 644 942 B1**
(45) Date of publication and mention of the grant of the patent: **25.01.2023**
(21) Application number: 18736835.2
(22) Date of filing: 26.06.2018
(51) Int. Cl.: A61K 8/25, A61K 8/26, A61Q 19/00, A61K 8/02, A61K 8/06, A61K 8/31

(54) **POWDER COSMETIC COMPOSITION BASED ON AN OIL-IN-WATER DISPERSION COATED WITH HYDROPHOBIC SILICA AEROGEL PARTICLES AND ALUMINA NANOPARTICLES**
PULVERFÖRMIGE KOSMETISCHE ZUSAMMENSETZUNG AUF DER BASIS EINER ÖL-IN-WASSER-DISPERSION, BESCHICHTET MIT HYDROPHOBEN SILICA-AEROGEL-TEILCHEN UND ALUMINIUMOXID-NANOPARTIKELN
COMPOSITION COSMÉTIQUE EN POUDRE À BASE D'UNE DISPERSION HUILE DANS L'EAU ENROBÉE DE PARTICULES HYDROPHOBES D'AÉROGEL DE SILICE ET DE NANOPARTICULES D'ALUMINE

(30) Priority: 26.06.2017 FR 1755816
(43) Date of publication of application: 06.05.2020
(73) Proprietor: L'Oreal, 75008 Paris (FR)
(72) Inventor: PINHEIRO BAIRRAS, Bernardino, 93601 Aulnay Sous Bois (FR)
(74) Representative: Lavoix
(86) International application number: PCT/EP2018/067089
(87) International publication number: WO 2019/002278

(56) References cited:
- WO-A1-2014/091012
- FR-A1- 2 992 170
- DOW CORNING: "Dow Corning VM-2270 Aerogel Fine particles", INTERNET CITATION, April 2009 (2009-04), pages 1-5, XP002650585, Retrieved from the Internet: URL:http://www2.dowcorning.com/DataFiles/0 90007c88020e235.pdf [retrieved on 2011-07-15]

## Description

The present invention relates to a cosmetic composition in powder form, comprising particles of hydrophobic silica aerogel and nanoparticles containing alumina, and a method of using said composition.

In the field of care products, it is known that compositions can be used in the form of creams, gels, or liquid formulas, for example of the serum type.

Such compositions are generally applied on dry skin, to achieve a homogeneous application and an optimal effect, for example a moisturizing effect.

Thus, the application of such compositions after a shower requires previous drying of the skin. Furthermore, these compositions can be difficult to spread (as is the case for example with formulas that are too fluid, or on the contrary are too thick). During transport, these compositions can partly escape from their packaging, which creates leaks.

Patent application WO2014/091012 describes cosmetic compositions in powder form comprising hydrophobic silica aerogel particles, which do not contain any oil. FR 2 992 170 discloses cosmetic compositions in the form of an oil-in-water emulsion comprising particles of hydrophobic silica aerogel. These particles of hydrophobic silica aerogel are notably sold by Dow Corning under the name VM-2270 (see Product Information Personal Care of Dow Corning, April 2009).

Therefore there is a need for compositions, and particularly care compositions, that are easy to apply, particularly on moist skin. Such compositions must also be easy to transport, lightweight and have no risk of leakage.

This invention responds to this need. It discloses a new galenic form of products, in the form of a powder. This powder, that can be called a "dry emulsion", is an oil-in-water emulsion comprising a minimum amount of water and coated with a specific coating. In use, this powder absorbs water present on the skin, particularly on the face and/or the body and/or the hair, particularly after a shower. It thus replaces the skin drying step, particularly using a towel, while providing care.

Such a composition is also easy to transport, without any risk of leaking. It is lightweight and takes up little space (it may be packaged in a small volume).

Therefore the purpose of this invention is a cosmetic composition in powder form, in which each powder grain is composed of:
- a dispersion of oily phase droplets in an aqueous phase, the oily phase being present in a content of at least 12% by weight of the total weight of the composition and comprising at least one mineral oil, and
- a coating, comprising at least some hydrophobic silica aerogel particles,
wherein the coating also comprises nanoparticles containing alumina.

This powder is suitable for application on moist or wet skin.

Another purpose of this invention is a method of preparing such a composition, comprising the mix of the oily phase, the aqueous phase and the coating, and optionally drying of the mix obtained. Preferably, the mixing step includes the use of a shear element.

In this description, the expression "at least one" is equivalent to the expression "one or several" and can be substituted for it.

In this description, the expression "included between" is equivalent to the expression "from ... to" and can be substituted for it.

The composition according to the invention is a powder. Each powder grain comprises a liquid core and a coating. The liquid core is a dispersion of oily phase droplets in an aqueous phase, i.e. an oil-in-water emulsion. The coating comprises at least some hydrophobic silica aerogel particles.

In other words, the composition according to the invention is a "dry" emulsion. Typically, the oil-in-water emulsion (making up the core) comprises an aqueous phase present in a minimum quantity, and is coated by at least some hydrophobic silica aerogel particles, preferably by a mixture of hydrophobic silica aerogel particles and nanoparticles containing alumina, in order to obtain a powder.

During use, in particular, the composition according to the invention absorbs water present on the face and/or body and/or hair, for example after a shower. The powder grains are then broken and mix with water; they can thus replace a towel for drying, and provide care at the same time.

Thus, the composition according to the invention is also called a "care powder".

As demonstrated through examples, the composition according to the invention has an innovative galenic form, and the silica aerogel particles of the coating, and the particular oily phase, play an important role in this galenic form.

### Oily phase

Compositions conforming with the invention comprise an oily phase dispersed in an aqueous phase. This oily phase is present in an amount of at least 12% by weight of the total weight of the composition, and comprises at least one mineral oil. The mineral oil is a mineral hydrocarbon oil.

For the purposes of this invention, "oily phase" denotes a phase comprising at least one oil and all liposoluble and lipophilic ingredients and fatty substances used for the formulation of compositions according to the invention.

Oil denotes any fatty substance in liquid form at ambient temperature (20 - 25°C) and at atmospheric pressure (760 mm Hg). An oil according to the invention may be volatile or non-volatile.

The composition comprises at least one mineral hydrocarbon oil. The composition may further comprise at least one additional oil. The additional oil may be chosen from hydrocarbon oils, silicon oils, fluorinated oils and mixtures thereof. A hydrocarbon oil suitable for the invention may be an animal hydrocarbon oil, a plant hydrocarbon oil or a synthetic hydrocarbon oil. The additional oil may be chosen from plant hydrocarbon oils, synthetic hydrocarbon oils, silicon oils and mixtures thereof.

For the purposes of this invention, the term "silicone oil" denotes an oil comprising at least one silicon atom, and in particular at least one SiO group.

The term "hydrocarbon oil" is intended to denote an oil containing mainly hydrogen and carbon atoms.

The term "fluorinated oil" denotes an oil comprising at least one fluorine atom.

A hydrocarbon oil according to the invention may also possibly comprise oxygen, nitrogen, sulfur and/or phosphorus atoms, for example in the form of hydroxyl, amine, amide, ester, ether or acid groups, and in particular in the form of hydroxyl, ester, ether or acid groups.

The oil may be volatile or non-volatile.

The term "volatile oil" according to the invention denotes any oil capable of evaporating in contact with skin or keratin fiber, in less than one hour, at ambient temperature and atmospheric pressure. The volatile oil(s) according to the invention is (are) volatile cosmetic oils that are liquid at ambient temperature, having a vapor pressure different from zero at ambient temperature and atmospheric pressure, particularly ranging from 0.13 Pato 40 000 Pa (10⁻³ to 300 mm Hg), particularly ranging from 1.3 Pa to 13 000 Pa (0.01 to 100 mm Hg), and more specifically ranging from 1.3 Pa to 1300 Pa (0.01 to 10 mm Hg).

The term "non-volatile oil" denotes an oil remaining on skin or keratin fiber at ambient temperature and atmospheric pressure for at least several hours and particularly having a vapor pressure less than 10⁻³ mm Hg (0.13 Pa).

### Hydrocarbon oils

Among non-volatile hydrocarbon oils that can be used according to the invention, mention may be made in particular of:
(i) hydrocarbon oils of plant origin such as glyceride triesters that are generally triesters of fatty acids and glycerols with fatty acid chain lengths ranging from C4 to C24, with the latter able to be linear or branched, saturated or unsaturated; these oils are in particular wheat germ, sunflower, grape seed, sesame, corn, apricot, castor, shea, avocado, olive, soybean oils, almond and particularly sweet almond, palm, rapeseed, cotton, hazelnut, macadamia, jojoba, alfalfa, poppy seed, pumpkin, sesame, squash, rapeseed, blackcurrant, evening primrose, millet, barley, quinoa, rye, safflower, candlenut, passiflora, musk rose, coconut oils; or caprylic/capric acid triglycerides such as those sold by Stearineries Dubois or those sold under the trade names Miglyol 810, 812 and 818 by Dynamit Nobel;
(ii) synthetic ethers having from 10 to 40 carbon atoms;
(iii) linear or branched hydrocarbons of mineral or synthetic origin, such as petrolatum oil, polydecenes, hydrogenated polyisobutene such as parleam, squalane, and mixtures thereof (mineral oils);
(iv) synthetic esters such as oils having the formula RCOOR' in which R represents the residue of a linear or branched fatty acid comprising 1 to 40 carbon atoms and R' represents a hydrocarbon chain, particularly branched containing 1 to 40 carbon atoms on the condition that R + R' is > 10, such as for example Purcellin oil (cetostearyl octanoate), isopropyl myristate, isopropyl palmitate, C12 to C15 alcohol benzoates like the product sold under the trade name "Finsolv TN" or "Witconol TN" by WITCO or "TEGOSOFT TN" by EVONIK GOLDSCHMIDT, 2-ethylphenyl benzoate such as the commercial product sold under the name "X-TEND 226" by ISP, isopropyl lanolate, hexyl laurate, diisopropyl adipate, isononyl isononanoate, oleyl erucate, 2-ethylhexyl palmitate, isostearyl isostearate, diisopropyl sebacate like the product sold under the trade name "Dub Dis" by Stearinerie Dubois, octanoates, decanoates or ricinoleates of alcohols or polyalcohols such as propylene glycol dioctanoate; hydroxylated esters, such as isostearyl lactate, diisostearyl malate; pentaerythritol esters; citrates or tartrates such as C12-C13 linear di-alkyl tartrates like those sold under the name COSMACOL ETI by ENICHEM AUGUSTA INDUSTRIALE and C14-C15 linear di-alkyl tartrates like those sold under the name COSMACOL ETL by the same company; acetates;
(v) fatty alcohols that are liquid at ambient temperature, with a branched and/or unsaturated carbon chain having 12 to 26 carbon atoms, such as octyldodecanol, isostearyl alcohol, oleic alcohol, 2-hexyldecanol, 2-butyloctanol, 2-undecylpentadecanol;
(vi) higher fatty acids, such as oleic acid, linoleic acid, linolenic acid;
(vii) carbonates such as dicaprylyl carbonate like the product sold under the name "Cetiol CC" by Cognis;
(viii) fatty amides such as isopropyl N-lauroyl sarcosinate like the product sold under the name Eldew SL-205 by Ajinomoto and mixtures thereof.

Preferably, the oily phase comprises at least one non-volatile mineral oil and at least one non-volatile plant hydrocarbon oil.

Preferably the non-volatile plant hydrocarbon oil is chosen from among glyceride triesters, and particularly triesters of fatty acids and glycerol wherein fatty acids have linear or branched, saturated or unsaturated chain lengths ranging from 4 to 24 carbon atoms.

More preferably, the non-volatile plant hydrocarbon oil is chosen from among wheat germ, sunflower, grape seed, sesame, corn, apricot, castor, shea, avocado, olive, soybean oils, almond and particularly sweet almond, palm, rapeseed, cotton, hazelnut, macadamia, jojoba, alfalfa, poppy seed, pumpkin, sesame, squash, rapeseed, blackcurrant, evening primrose, millet, barley, quinoa, rye, safflower, candlenut, passiflora, musk rose, coconut oils; or caprylic/capric acid triglycerides.

Volatile hydrocarbon oils that can be used according to the invention can be chosen particularly from among hydrocarbon oils having 8 to 16 carbon atoms, and particularly branched C8-C16 alkanes such as petroleum-based C8-C16 isoalkanes (also referred to as isoparaffins) such as isododecane (also referred to as 2,2,4,4,6-pentamethylheptane), isodecane, isohexadecane, oils sold under the trade names Isopars or Permetyls, branched C8-C16 esters, iso-hexyl neopentanoate, and mixtures thereof.

Mention can also be made of the alkanes described in patent applications WO 2007/068371, or WO2008/155059 made by Cognis (separate alkane mixtures and different by at least one carbon). These alkanes are obtained from fatty alcohols, themselves obtained from coconut or palm oil. Mention can be made of mixtures of n-undecane (C11) and n-tridecane (C13) obtained in examples 1 and 2 in the application WO2008/155059 made by Cognis.

Mention can also be made of n-dodecane (C12) and n-tetradecane (C14) sold by Sasol under references PARAFOL 12-97 and PARAFOL 14-97 respectively, and mixtures thereof.

Further volatile hydrocarbon oils such as petroleum distillates, particularly those sold under the name Shell Solt by SHELL, may also be used.

In one embodiment, the volatile solvent is chosen from among volatile hydrocarbon oils having 8 to 16 carbon atoms and mixtures thereof.

### Silicone oils

The non-volatile silicone oils can be chosen particularly from among non-volatile polydimethylsiloxanes (PDMS), polydimethylsiloxanes comprising alkyl or alkoxy groups that are pendant and/or at the end of the silicone chain, groups each having 2 to 24 carbon atoms; phenylated silicones, such as phenyl trimethicones, phenyl dimethicones, phenyltrimethylsiloxydiphenylsiloxanes, diphenyl dimethicones, diphenylmethyldiphenyl-trisiloxanes or (2-phenylethyl)trimethylsiloxysilicates.

Volatile silicone oils that can be used include for example volatile linear or cyclic silicone oils, particularly those having a viscosity of 8 centistokes (8 × 10⁻⁶ m²/s), and in particular having 2 to 7 silicon atoms, these silicones optionally comprising alkyl or alkoxy groups having 1 to 10 carbon atoms. Mention may be made, as a volatile silicone oil suitable for use in the invention, in particular, of octamethyl cyclotetrasiloxane, decamethyl cyclopentasiloxane, dodecamethyl cyclohexasiloxane, heptamethyl hexyltrisiloxane, heptamethyloctyl trisiloxane, hexamethyl disiloxane, octamethyl trisiloxane, decamethyl tetrasiloxane, dodecamethyl pentasiloxane, and mixtures thereof.

Mention may also be made of volatile alkyl trisiloxanes oils with general formula (I):

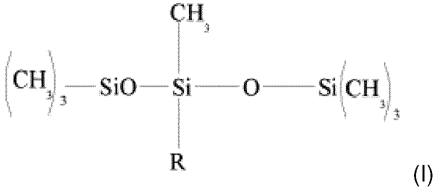

In which R represents an alkyl group comprising 2 to 4 carbon atoms and in which one or several hydrogen atoms can be substituted by a fluorine or chlorine atom.

Among oils with general formula (I), mention may be made of: 3-butyl 1,1,1,3,5,5,5-heptamethyl trisiloxane, 3-propyl 1,1,1,3,5,5,5-heptamethyl trisiloxane, and 3-ethyl 1,1,1,3,5,5,5-heptamethyl trisiloxane, corresponding to oils with formula (I) for which R is a butyl group, a propyl group or an ethyl group respectively.

Preferably, the oily phase comprises at least one silicone oil, preferably a non-volatile PDMS.

### Fluorinated oils

It would also be possible to use volatile fluorinated oils such as nonafluoromethoxybutane, nonafluoromethoxybutane, decafluoropentane, tetradecafluorohexane, dodecafluoropentane and mixtures thereof.

The oily phase may also comprise other fatty substances. Another fatty substance (or lipophilic compound) that can be present in the oily phase can be chosen from among:
- fatty acids comprising 8 to 30 carbon atoms such as stearic acid, lauric acid, palmitic acid and oleic acid;
- waxes such as lanolin, beeswax, Carnauba or Candellila wax, paraffin or lignite waxes or microcrystalline waxes, ceresin or ozokerite, synthetic waxes such as polyethylene waxes and Fischer-Tropsch waxes;
- pasty compounds, detailed below, such as butters of plant origin;
- fatty alcohols solid at ambient temperature with a linear carbon chain having 12 to 26 carbon atoms such as cetylstearylic alcohol;
- and mixtures thereof.

More preferably, the oily phase also comprises at least one lipophilic compound chosen from among fatty alcohols solid at ambient temperature with a linear carbon chain with 12 to 26 carbon atoms, silicone oils, fatty acids comprising 8 to 30 carbon atoms, waxes, pasty compounds, particularly as defined below and mixtures thereof.

Even more preferably, the oily phase also comprises a mix of a fatty alcohol solid at ambient temperature with a linear carbon chain having 12 to 26 carbon atoms, a silicone oil, a fatty acid comprising 8 to 30 carbon atoms, a wax and a pasty compound (particularly as defined below).

As mentioned above, the composition according to the invention may comprise at least one pasty hydrocarbon or silicon compound (or pasty fatty substance) at 23°C.

For the purposes of the invention, the term "pasty fatty substance" refers to a lipophilic fatty compound having a reversible solid/liquid change of state, having an anisotropic crystalline organization in the solid state, and comprising a liquid fraction and a solid fraction at a temperature of 23°C.

In other words, the initial melting point of the pasty compound may be less than 23°C. The liquid fraction of the pasty compound measured at 23°C may represent 9 to 97% by weight of the compound. This liquid fraction at 23°C preferably represents between 15 and 85%, more preferably between 40 and 85% by weight.

The melting point of a solid fatty substance can be measured using a differential scanning calorimeter (DSC), for example the calorimeter sold under the trade name "DSC Q100" by TA Instruments with the "TA Universal Analysis" software, according to the protocol defined hereinabove.

The liquid fraction by weight of the pasty compound at 23°C is more particularly equal to the ratio of the enthalpy of fusion consumed at 23°C to the enthalpy of fusion of the pasty compound.

The enthalpy of fusion of the pasty compound is the enthalpy consumed by the compound to change from the solid state to the liquid state. The pasty compound is said to be in the solid state when the entire mass thereof is in solid crystalline form. The pasty compound is said to be in the liquid state when the entire mass thereof is in liquid form.

The enthalpy of fusion of the pasty compound is in particular equal to the area under the curve of the thermogram obtained using a differential scanning calorimeter. The enthalpy of fusion of the pasty compound is the quantity of energy required to change the compound from the solid state to the liquid state. It is expressed in J/g.

The enthalpy of fusion consumed at 23°C is the quantity of energy required by the sample to change from the solid state to the state presented at 23°C consisting of a liquid fraction and a solid fraction.

The pasty compound(s) can be in particular chosen from synthetic pasty compounds and fatty substances of plant origin. The pasty compound(s) can be hydrocarbon or silicone.

The pasty compound(s) can be in particular chosen from:
- lanolin and its derivatives, such as lanolin alcohol, oxyethylenated lanolins, acetylated lanolin, lanolin esters such as isopropyl lanolate, oxypropylenated lanolins;
- vaseline (also called petrolatum),
- polyol ethers chosen from pentaerythritol and C2-C4 polyalkylene glycol ethers, fatty alcohol and sugar ethers, and mixtures thereof. For example, mention can be made of the ether of pentaerythritol and polyethylene glycol comprising 5 oxyethylene patterns (5 OE) (CTFA name: PEG-5 Pentaerythrityl Ether), the ether of pentaerythritol and polypropylene glycol comprising 5 oxypropylene units (5 OP) (CTFA name: PPG-5 Pentaerythrityl Ether), and the mixtures thereof and more specifically the mixture of PEG-5 Pentaerythrityl Ether, PPG-5 Pentaerythrityl Ether and soybean oil, sold under the name "Lanolide" by VEVY, wherein the ratio of the constituents by weight is 46:46:8: 46% PEG-5 Pentaerythrityl Ether, 46% PPG-5 Pentaerythrityl Ether and 8% soybean oil,
- oligomer glycerol esters, especially the esters of diglycerol, with monocarboxylic acids, possibly hydroxylated, linear or branched, saturated or not, preferably saturated, C6-C20, and/or dicarboxylic acids, linear or branched, saturated or not, preferably saturated, C6-C10, in particular condensates of adipic acid and diglycerol, for which a portion of the hydroxyl groups of the glycerols have reacted with a mixture of fatty acids, such as stearic acid, capric acid, stearic acid, isostearic acid and 12-hydroxystearic acid, such as for example bis-diglyceryl polyacyladipate-2 sold under the reference SOFTISAN^{®} 649 by Sasol,
- homopolymers of vinyl ester having C8-C30 alkyl groups, such as polyvinyl laurate (in particular sold under the reference Mexomere PP by Chimex),
- arachidyl propionate sold under the brand Waxenol 801 by ALZO,
- phytosterol esters,
- triglycerides of fatty acids and their derivatives, in particular triglycerides of fatty acids, saturated or not, linear or branched, possibly mono or poly hydroxylated, C6-C30, more particularly C8-C18, possibly hydrogenated (totally or partially); with for example Softisan 100^{®} sold by Sasol,
- pentaerythritol esters,
- aliphatic esters derived from esterification of an aliphatic hydroxycarboxylic acid with an aliphatic carboxylic acid. More particularly, the aliphatic carboxylic acid is C₄-C₃₀, preferably C₈-C₃₀. It is preferably chosen from hexanoic, heptanoic, octanoic, 2-ethylhexanoic, nonanoic, decanoic, undecanoic, dodecanoic, tridecanoic, tetradecanoic, pentadecanoic, hexadecanoic, hexyldecanoic, heptadecanoic, octadecanoic, isostearic, nonadecanoic, eicosanoic, isoarachidic, octyldodecanoic, heneicosanoic and docosanoic acids, and mixtures thereof. The aliphatic carboxylic acid is preferably branched. The hydroxycarboxylic acid ester is advantageously derived from a C2-C40 hydroxylated carboxylic acid, preferably C10 - C34, and more preferably C12 - C28; with the number of hydroxyl groups being between 1 and 20, more particularly between 1 and 10, preferably between 1 and 6,
- dimer diol and dimer diacid esters, optionally esterified on the alcohol or free acid function(s) thereof by acid or alcohol radicals, in particular dilinoleic dimer esters; such esters may particularly be chosen from esters having the following INCI classification: bis-behenyl/isostearyl/phytosteryl dimer dilinoleyl dimer dilinoleate (Plandool G), phytosteryl/isosteryl/cetyl/stearyl/behenyl dimer dilinoleate (Plandool H or Plandool S) and mixtures thereof,
- hydrogenated rosin esters (Lusplan DD-DHR or DD-DHR from Nippon Fine Chemical)
- butters of plant origin, such as mango butter, such as the one sold under the reference Lipex 203 by AARHUSKARLSHAMN, shea butter, in particular the one of which the INCI name is Butyrospermum Parkii Butter, such as the one sold under the reference Sheasoft^{®} by AARHUSKARLSHAMN, cupuacu butter (Rain forest RF3410 from Beraca Sabara), murumuru butter (RAIN FOREST RF3710 from Beraca Sabara), cocoa butter, babassu butter such as the one sold under the name Cropure Babassu SS-(LK) by Croda, as well as orange wax such as, for example, the one sold under the reference Orange Peel Wax by Koster Keunen,
- totally or partially hydrogenated plant oils, such as for example hydrogenated soybean oil, hydrogenated coconut oil, hydrogenated rapeseed oil, mixtures of hydrogenated plant oils such as the mixture of hydrogenated plant oils of soybean, coconut, palm and rapeseed, for example the mixture sold under the reference Akogel^{®} by AARHUSKARLSHAMN (INCI name Hydrogenated Vegetable Oil), trans isomerized partially hydrogenated jojoba oil manufactured or sold by Desert Whale under the commercial reference Iso-Jojoba-50^{®}, partially hydrogenated olive oil such as, for example, the compound sold under the reference Beurrolive by Soliance,
- hydrogenated castor oil esters, such as dimer dilinoleate hydrogenated castor oil for example RISOCAST-DA-L sold by KOKYU ALCOHOL KOGYO, hydrogenated castor oil isostearate for example SALACOS HCIS (V-L) sold by NISSHIN OIL,
- and mixtures thereof.

Preferably, the pasty compound(s) that can be used with the invention is (are) chosen from among butters of plant origin, preferably those mentioned above.

According to one embodiment, the composition comprises from 0.1 to 5% by weight, preferably from 0.2 to 3%, and even better from 0.3 to 1.5% by weight of pasty compound, relative to the total weight of the composition.

Preferably, the oily phase content is between 13% and 40% by weight, preferably between 13.5% and 35% by weight relative to the total weight of the composition.

### Aqueous phase

The compositions according to the invention comprise at least one aqueous phase.

The aqueous phase contains water and possibly at least one organic solvent that is soluble or miscible in water.

An aqueous phase suitable for the invention may for example comprise water chosen from among a natural spring water, for example such as La Roche-Posay water, Vittel water, or Vichy water, or a floral water.

Preferably, the water content is between 5% and 70% by weight of the total weight of the composition, preferably between 6% and 60% by weight.

The water-soluble or water-miscible solvents suitable for the invention comprise short-chain mono-alcohols for example C1-C4 such as ethanol, isopropanol; diols or polyols such as ethylene glycol, 1 ,2-propylene glycol, 1 ,3-butylene glycol, hexylene glycol, diethylene glycol, dipropylene glycol, 2-ethoxyethanol, diethylene glycol monomethylether, triethylene glycol monomethylether, glycerol, sorbitol and mixtures of them.

According to one preferred embodiment, in particular ethanol, propylene glycol, glycerine or one of their mixtures can be used.

Preferably, the content of water-soluble or water-miscible solvent is between 4% and 20% by weight of the total weight of the composition, preferably between 5% and 15% by weight.

Preferably according to one particular form of the invention, the content of the aqueous phase is between 10% and 75% by weight of the total weight of the composition, preferably between 15% and 70% by weight.

### Aerogel particles of hydrophobic silica

The composition (powder) according to the invention comprises a coating comprising at least some hydrophobic silica aerogel particles.

Aerogels are ultra-lightweight porous materials, the first of which were made by Kristler in 1932.

They are generally synthesized by a sol-gel method in a liquid medium then dried by extraction of a supercritical fluid. The most commonly used supercritical fluid is supercritical CO2. This type of drying makes it possible to prevent contraction of the pores and of the material. There are other types of drying that can also be used to obtain porous materials starting from a gel, for example (i) drying by cryodessiccation, consisting of solidifying the gel at low temperature and then sublimating the solvent and (ii) drying by evaporation. The materials thus obtained are called cryogels and xerogels respectively. The sol-gel method and the various dryings are described in detail in Brinker CJ., and Scherer G.W., Sol-Gel Science: New York: Academic Press, 1990.

"Hydrophobic silica" denotes any silica for which the surface is treated by silylation agents, for example by halogenated silanes such as alkylchlorosilanes; silanes and particularly dimethylsiloxanes such as hexamethyldisiloxane; or silazanes, so as to functionalize OH groups by Si-Rn silyl groups, for example trimethylsilyl groups.

Preferably, aerogel particles of hydrophobic silica that could be used in this invention advantageously have a specific area per unit mass (SM) varying from 500 to 1500 m²/g, preferably from 600 to 1200 m²/g and even better from 600 to 800 m²/g.

Preferably, aerogel particles of hydrophobic silica that could be used in this invention advantageously have an oil absorption capacity measured at the WET POINT varying from 5 to 18 ml/g of particles, preferably from 6 to 15 ml/g and better from 8 to 12 ml/g.

Preferably, aerogel particles of hydrophobic silica that could used in this invention advantageously have a size, expressed as an average diameter (D[0.5]), less than 1500 µm and preferably varying from 1 to 30 µm, preferably from 5 to 25 µm, even better from 5 to 20 µm and even better again from 5 to 15 µm.

Preferably, aerogel particles of hydrophobic silica that could used in this invention advantageously have a compacted density ρ varying from 0.04 g/cm³ to 0.10 g/cm³, and preferably from 0.05 g/cm³ to 0.08 g/cm³.

Preferably, aerogel particles of hydrophobic silica that could be used in this invention advantageously have a specific area per unit volume (SV) varying from 5 to 60 m²/cm³, preferably from 10 to 50 m²/cm³ and even better from 15 to 40 m²/cm³.

According to one preferred embodiment, the aerogel particles of hydrophobic silica according to the invention have a specific area per unit mass (SM) varying from 500 to 1500 m²/g, preferably from 600 to 1200 m²/g and even better from 600 to 800 m²/g, and a size expressed as an average diameter (D[0.5]) varying from 1 to 30 µm and/or an oil absorption capacity measured at the WET POINT varying from 5 to 18 ml/g of particles, preferably 6 to 15 ml/g and even better from 8 to 12 ml/g.

According to another preferred embodiment, the aerobic particles of hydrophobic silica used in this invention have a specific area per unit mass (SM) varying from 600 à 800 m²/g and a size expressed as an average diameter in volume (D[0.5]) varying from 5 to 20 µm, and better from 5 to 15 µm.

The specific area per unit mass may be determined using the nitrogen absorption method called the BET (BRUNAUER - EMMET - TELLER) method described in "The journal of the American Chemical Society", vol. 60, page 309, February 1938 and corresponding to international standard ISO 5794/1 (Appendix D). The BET specific area is the total specific area of the particles considered.

The absorption capacity measured at the WET POINT and denoted Wp, corresponds to the quantity of oil that must be added to 100 g of particles in order to obtain a homogeneous paste. It is measured using the Wet Point method or the method for determining oil take-up of powder according to the principle described in standard NF T 30-022. It corresponds to the quantity of oil adsorbed on the available surface of the powder and/or absorbed by the powder according to the Wet Point measurement, as described below:
A quantity m = 2 g of powder is placed on a glass plate and oil (isononyl isononanoate) is then added drop by drop. After adding 4 to 5 drops of oil into the powder, it is mixed using a spatula and the addition of oil is continued until oil and powder conglomerates are formed. After this stage, oil is added one drop at a time and the mixture is then triturated with the spatula. The addition of oil is stopped when a firm and smooth paste is obtained. This paste should be allowed to spread on the glass plate without crazing and without the formation of lumps. The volume Vs (expressed in ml) of oil used is then noted.

The oil take-up (oil absorption capacity) corresponds to the Vs / m ratio.

The sizes of aerogel particles according to the invention can be measured by static diffusion of light by means of a commercial Malvern MasterSizer 2000 granunlometer. Data are processed based on Mie's scattering theory. This theory, precise for isotropic particles, can determine an "effective" particle diameter in the case of non-spherical particles. This theory is described particularly in the book by Van de Hulst, H.C., "Light Scattering by Small Particles", Chapters 9 and 10, Wiley, New York, 1957.

In the framework of this invention, the compacted density can be evaluated using the following protocol called the compacted density protocol: 40 g of powder is poured into a graduated test tube and the test tube is then placed on a STAV 2003 apparatus made by STAMPF VOLUMETER. A series of 2500 compactions is then applied to the test tube (this operation is repeated until the difference in volume between two successive compactions is less than 2%); the final volume Vf of compacted powder is then measured on the test piece directly. The compacted density is then determined by the mass(m)A/f, ratio, in fact 40/Vf (where Vf is expressed in cm³ and m is expressed in g).

The specific area per unit volume is given by the relation: SV = SM × p, in which p is the compacted density expressed in g/cm³ and SM is the specific area per unit mass expressed in m²/g, as defined above.

Aerogel particles of hydrophobic silica used according to this invention are preferably aerogel particles of silica silylate (INCI name).

The preparation of aerogel particles of hydrophobic silica modified on the surface by silylation is described earlier in document US 7,470,725.

Particles of hydrophobic silica aerogels modified on the surface by trimethylsilyl groups will be used in particular.

Hydrophobic silica aerogels that can be used within the invention include for example the aerogel sold under the name VM-2260 (INCI name Silica silylate), by Dow Corning, of which the particles have an average size of about 1000 microns and a specific area per unit mass ranging from 600 to 800 m²/g.

Mention can also be made of the aerogels sold by Cabot under the references AEROGEL TLD 201, AEROGEL OGD 201 and AEROGEL TLD 203, ENOVA AEROGEL MT 1100, ENOVA AEROGEL MT 1200.

In particular, the aerogel sold under the name VM-2270 (INCI name Silica silylate), by Dow Corning, of which the particles have an average size ranging from about 5 to 15 microns and a specific area per unit mass ranging from 600 to 800 m²/g, will be used.

Obviously, a mix of hydrophobic silia aerogel particles can be used.

Compositions according to the invention can include a quantity of hydrophobic silica aerogel particles equal to between 0.01 and 5% by weight, preferably between 0.1 and 3% by weight, and even better between 0.2 and 1% by weight in relation to the total weight of the composition.

### Nanoparticles containing alumina

The composition according to the invention also contains nanoparticles containing alumina.

"Nanoparticles" refers to solid particles with an average primary size in number between 5 and 25 nm, and preferably between 8 and 18 nm.

According to this invention, solid nanoparticles containing alumina form a mass in which alumina does not act as a coating, encapsulation or adsorption agent for one or other fillers, such as one or more metal oxides.

If the nanoparticles are formed by alumina and other fillers, the alumina is in the free state and does not form chemical bonds with other fillers. It is then an alloy between alumina and other fillers, particularly with metal or metalloid oxides (chosen particularly from among silicon or boron oxide), and particularly obtained by thermal fusion of these different constituents.

In the meaning of this invention, "primary particle size" means the maximum dimension that can be measured between two diametrically opposite points on the particle. The average primary size in number can be determined either by electron transmission microscopy, or by measurement of the specific area using the BET technique.

Alumina nanoparticles can for example have an arbitrary shape, for example they may be in the form of spheres, flakes, needles or completely random shapes.

Preferably, the nanoparticles contain at least 50% alumina.

According to a more advantageous embodiment of this invention, the nanoparticles are alumina nanoparticles.

Advantageously, the nanoparticles containing alumina are present in the composition with a concentration by weight relative to the total weight of the composition equal to between 15% and 60%, and preferably between 17% and 50%, and even more preferably between 18 and 45% by weight.

Preferably, the nanoparticles containing alumina are used in the native state, in other words without being encapsulated or coated by any agent whatsoever such as a polymer.

Preferably, the nanoparticles containing alumina are marketed by Sensient under the trade name ALUMINA AS-EM.

Advantageously, the weight ratio (hydrophobic silica aerogel particles): (nanoparticles containing alumina) is between 0.005 and 0.03, and preferably between 0.01 and 0.02.

Preferably, the composition according to the invention comprises the following by weight relative to the total weight of the composition:
- from 55% to 70% of aqueous phase;
- from 10% to 20% by weight of oily phase; and,
- from 15% to 25% by weight of coating comprising hydrophobic silica aerogel particles and nanoparticles containing alumina.

Preferably, the composition according to the invention comprises the following by weight relative to the total weight of the composition:
- from 15% to 40% of aqueous phase;
- from 20% to 35% by weight of oily phase; and,
- from 30% to 50% by weight of coating comprising hydrophobic silica aerogel particles and nanoparticles containing alumina.

Preferably, the composition according to the invention comprises the following by weight relative to the total weight of the composition:
- from 30% to 60% of aqueous phase;
- from 15% to 25% by weight of oily phase; and,
- from 20% to 40% by weight of coating comprising hydrophobic silica aerogel particles and nanoparticles containing alumina.

Compositions according to this invention may also include classical cosmetic additives, chosen particularly from among thickeners, perfumes; preservatives, surfactants, active constituents and particularly moisturizing agents, fillers, alkanizing agents or acidifiers or any other ingredient usually used for cosmetics and/or dermatology.

Thickeners include carboxyvinyl polymers such as Carbopols^{®} (Carbomers) and Pemulens such as Pemulen TR1^{®} and Pemulen TR2^{®} (acrylate/C10-C30 alkylacrylate crosspolymer); polyacrylamides such as for example cross-linked copolymers sold under the names Sepigel 305^{®} (C.T.F.A. name: : polyacrylamide/C13-14 isoparaffin/Laureth 7) or Simulgel 600 (C.T.F.A. name : acrylamide / sodium acryloyldimethyltaurate copolymer / isohexadecane / polysorbate 80) by Seppic; 2-acrylamido 2-methylpropane sulfonic acid polymers and copolymers, optionally cross-linked and/or neutralized, such as poly(2-acrylamido 2-methylpropane sulfonic acid) marketed by Hoechst under the trade name "Hostacerin AMPS^{®}" (CTFA name: ammonium polyacryloyldimethyl taurate or SIMULGEL 800^{®} marketed by SEPPIC (CTFA name: sodium polyacryolyldimethyl taurate / polysorbate 80 / sorbitan oleate); 2-acrylamido-2-methylpropane sulfonic acid and hydroxyethyl acrylate copolymers such as SIMULGEL NS^{®} and SEPINOV EMT 10^{®} sold by SEPPIC; cellulose derivatives such as hydroxyethylcellulose; polysaccharides and in particular gums such as xanthan gum; water-soluble or water-dispersible silicone derivatives such as acrylic silicones, silicone polyethers and cationic silicones and mixtures thereof.

The thickeners are preferably present in a quantity between 0.1% and 5% by weight, and preferably between 0.2% and 1% by weight in relation to the total weight of the composition.

Surfactants are preferably chosen from among anionic, cationic, non-ionic, zwitterionic and amphoteric surfactants. Preferably, they are chosen from among:
a) non-ionic surfactants, and particularly with a HLB at or above 8 to 25°C, used alone or in a mixture. Mention can be made in particular of:
   esters and ethers of oses such as the mixture of cetylstearyl glucoside and cetyl and stearyl alcohols such as Montanov 68 from Seppic;
   oxyethylene and/or oxypropylene ethers (that may comprise from 1 to 150 oxyethylene and/or oxypropylene groups) of glycerol;
   oxyethylene and/or oxypropylene ethers (that may comprise from 1 to 150 oxyethylene and/or oxypropylene groups) of fatty alcohols (particularly C8-C24 and preferably C12-C18 alcohols) such as oxyethylene ether of cetearylic alcohol with 30 oxyethylene groups (CTFA name "Ceteareth-30"), oxyethylene ether of stearylic alcohol with 20 oxyethylene groups (CTFA name "Steareth-20"), oxyethylene ether of the mix of C12-C15 fatty alcohols containing 7 oxyethylene groups (CTFA name "C12-15 Pareth-7") marketed particularly under the name NEODOL 25-7^{®} by SHELL CHEMICALS;
   esters of polyoxyalkylated fatty acids (particularly polyoxyethylenated and/or polyoxypropylenated) possibly in association with a fatty acid ester and glycerol such as the PEG-100 Stearate/Glyceryl Stearate mixture marketed for example by Croda under the trade name Arlacel 165;
   fatty acid esters (particularly C8-C24 acid, and preferably C16-C22 acid) and oxyethylenated and/or oxypropylated glycerol ethers (that may include 1 to 150 oxyethylanated and/or oxypropylenated groups), such as PEG-200 glyceryl monostearate sold particularly under the name Simulsol 220 TM^{®} by SEPPIC; PEG-50 stearate and PEG-40 monostearate marketed particularly under the name MYRJ 52P^{®} by ICI UNIQUEMA; polyethoxylated glyceryl stearate with 30 ethylene oxide groups such as the TAGAT S^{®} product sold by Evonik GOLDSCHMIDT, polyethoxylated glyceryl oleate with 30 ethylene oxide groups like the TAGAT O^{®} product sold by Evonik GOLDSCHMIDT, polyethoxylated glyceryl cocoate with 30 ethylene oxide groups like the VARIONIC LI 13^{®} product sold by SHEREX, polyethoxylated glyceryl isostearate with 30 ethylene oxide groups such as the TAGAT L^{®} product sold by Evonik GOLDSCHMIDT and polyethoxylated glyceryl laurate with 30 groups of ethylene oxide like the TAGAT I^{®} product from Evonik GOLDSCHMIDT,
   fatty acid esters (particularly C8-C24 acid and preferably C16-C22 acid) and oxyethylenated and/or oxypropylenated sorbitol ethers (possibly containing 1 to 150 oxyethylenated and/or oxypropylenated groups), such as polysorbate 20 particularly sold under the name Tween 20^{®} by CRODA, polysorbate 60 particularly sold under the name Tween 60^{®} by CRODA,
   dimethicone copolyol, like that sold under the name Q2-5220^{®} by DOW CORNING,
   dimethicone copolyol benzoate (FINSOLV SLB 101^{®} and 201^{®} from FINTEX),
      - copolymers of propylene oxide and of ethylene oxide, also called OE/OP polycondensates,
   and mixtures thereof.
b) anionic surfactants such as:
   polyoxyethylenated fatty acid salts and particularly those derived from amines or alkaline salts, and mixtures of them;
   phosphoric esters and their salts such as "DEA oleth-10 phosphate" (Crodafos N 10N from CRODA) or monopotassium monocetyl phosphate or potassium cetyl phosphate (Amphisol K from Givaudan);
   sulfosuccinates such as "Disodium PEG-5 citrate lauryl sulfosuccinate" and "Disodium ricinoleamido MEA sulfosuccinate";
   alkylethersulfates such as sodium lauryl ether sulfate;
   isethionates;
   acylglutamates such as "Disodium hydrogenated tallow glutamate" (AMISOFT HS-21 R^{®} marketed by AJINOMOTO) and sodium stearoyl glutamate (AMISOFT HS-11 PF^{®} marketed by AJINOMOTO) and mixtures of them;
   derivatives of soybeans such as potassium soyate;
   citrates, such as Glyceryl stearate citrate (Axol C 62 Pellets from Degussa);
   derivatives of proline, such as Sodium palmitoyl proline (Sepicalm VG from Seppic), or the Mixture of Sodium palmitoyl sarcosinate, Magnesium palmitoyl glutamate, palmitic acid and Palmitoyl proline (Sepifeel One from Seppic) ;
   lactylates, such as Sodium stearoyl lactylate (Akoline SL from Karlshamns AB) ;
   sarcosinates, such as sodium palmitoyl sarcosinate (Nikkol sarcosinate PN) or the mixture of Stearoyl sarcosine and Myristoyl sarcosine 75/25 (Crodasin SM from Croda) ;
   sulfonates, such as Sodium C14-17 alkyl sec sulfonate (Hostapur SAS 60 from Clariant) ;
   glycinates, such as sodium cocoyl glycinate (Amilite GCS-12 from Ajinomoto).

Preferably, the surfactant used is a non-ionic surfactant with a HLB greater than or equal to 8 to 25°C.

The surfactant is preferably present in a quantity between 0.1% and 5% by weight, preferably between 0.2% and 3% by weight, preferably between 0.5% and 2% by weight, in relation to the total weight of the composition.

Another purpose of this invention is a method of preparing such a composition, comprising the mixture of the oily phase, the aqueous phase and the coating, and optionally drying of the mixture obtained. Preferably, the mixing step includes the use of a shear element. Preferably, the mixing step is done by short term stirring, i.e. for a few seconds, for example from 10 to 60 seconds.

Such a method is illustrated in the examples.

The invention will be better understood after studying the following non-limitative examples that form preferred embodiments of the method conforming with the invention.

Unless mentioned otherwise, quantities in the following examples are expressed as a percent by weight.

### Example 1:

The following compositions are prepared:

| **INCI name** | **Formula according to the invention** | **Comparative formulas** | | |
|---|---|---|---|---|
| | | **A** | **B** | **C** |
| Water | Qsp100 | Qsp100 | Qsp100 | Qsp100 |
| Glycerin | 7 | 5 | 10 | 7 |
| Sodium hydroxide | 0.1 | | | 0.11 |
| Glyceryl stearate and PEG-100 stearate | 1 | | 1.4 | 1.7 |
| SODIUM STEAROYL GLUTAMATE | | 0.2 | | |
| MYRISTAMIDOPROPYL PG-DIMONIUM CHLORIDE PHOSPHATE | | 0.5 | | |
| CETEARYL GLUCOSIDE | | 0.5 | | |
| GLYCERYL STEARATE | | | 2.8 | |
| MYRETH-3 MYRISTATE | | | | 2 |
| fatty acids | 0.5 | | | |
| petrolatum oil | 8.25 | | | |
| almond oil | 0.01 | 0.5 | 0.1 | |
| cetylstearylic alcohol | 4 | | 4.3 | |
| avocado oil | 0.01 | | | |
| jojoba oil | 0.01 | | | |
| shea butter | 0.6 | 0.5 | 0.5 | |
| apricot oil | 0.01 | | | |
| paraffin oil | 4.5 | | | |
| coconut oil | 0.01 | | | |
| SESAMUM INDICUM (SESAME) SEED OIL | | 1.75 | | |
| GLYCINE SOJA (SOYBEAN) OIL | | 0.5 | | |
| ORBIGNYA OLEIFERA SEED OIL | | 0.5 | | |
| CETEARYL ALCOHOL | | 0.5 | | |
| CAPRYLIC/CAPRIC TRIGLYCERIDE | | 2.5 | | |
| PENTAERYTHRITYL DISTEARATE | | 0.7 | | |
| ISOPROPYL PALMITATE | | | 5 | 2 |
| CETYL ESTERS (and) CETYL ESTERS | | | 1.2 | |
| OCTYLDODECANOL | | | | 1.5 |
| PENTAERYTHRITYL TETRAETHYLHEXANOATE | | | | 3 |
| CETYL ALCOHOL | | 1 | | 2 |
| STEARYL ALCOHOL | | | | 0.5 |
| carbomer | 0.3 | | | 0.6 |
| XANTHAN GUM | | 0.15 | | |
| SODIUM POLYACRYLATE | | 1.1 | 0.28 | |
| METHYL METHACRYLATE CROSSPOLYMER | | | | 0.8 |
| SYNTHETIC WAX | | | | 1 |
| polydimethysiloxane | 0.3 | | 2.5 | |
| DIMETHICONE | | | | 1.5 |
| DIMETHICONE (and) DIMETHICONE/VINYL DIMETHICONE CROSSPOLYMER | | | | 2 |
| DIMETHICONE CROSSPOLYMER | | | | 2 |
| CAPRYLYL GLYCOL | | 0.5 | 0.25 | 0.35 |
| Ethanol | | | | 3 |
| PENTYLENE GLYCOL | | | | 2 |
| TOCOPHEROL | 0.1 | | | 0.5 |
| HONEY | | 1 | | |
| PRUNUS AMYGDALUS DULCIS (SWEET ALMOND) SEED EXTRACT | | 0.05 | | |
| MAGNESIUM ALUMINUM SILICATE (and) PRUNUS AMYGDALUS DULCIS (SWEET ALMOND) FRUIT EXTRACT (and) XANTHAN GUM | | | 0.1 | |
| AVENA SATIVA (OAT) KERNEL EXTRACT | | | 0.1 | |
| VACCINIUM MYRTILLUS FRUIT EXTRACT | | | | 2 |
| TITANIUM DIOXIDE (and) MICA | | | | 0.4 |
| FRAGRANCE | | 0.6 | 0.6 | 0.5 |
| CAPRYLOYL SALICYLIC ACID | | | | 0.3 |
| SACCHAROMYCES/XYLINUM/BLACK TEA FERMENT (and) GLYCERIN | | | | 3 |
| Lysate of lactic ferment | 0.01 | | | |
| DISODIUM EDTA | | 0.05 | | 0.15 |
| ADENOSINE | | | | 0.1 |
| SILICA | | | | 0.5 |
| Colorants | 0.00206 | | 0.00015 | 0.00035 |
| Preservative | Qs | Qs | Qs | Qs |
| **Oily phase quantity** | **18** | **8.5** | **13.6** | **11.5** |

### Procedure:

80% of formula (according to the invention or comparative) with a mix of 0.26% of silica aerogel and 19.74% of alumina is weighed directly in the packaging article. Stirring is done slowly by shaking the packaging article manually for about 10 seconds. The pneumatic vibrator is activated on the packaging article and shaking is continued for 30 seconds, which is about 80 pulses.

The results obtained show that the formula according to the invention is the formula that obtains a powder in which each grain is composed of an oil-in-water emulsion coated with the mix of silica aerogel and alumina.

The 3 comparative formulas A, B and C mixed with silica aerogel and alumina give a cream and not a powder.

### Example 2:

### Procedure:

The mixture (0.26% of silica aerogel or hydrophobic fumed silica, and 19.74% of alumina) and then 80% of cream (formula according to the invention or comparative) is weighed directly in the packaging article. Stirring is done slowly by shaking the packaging article manually for about 10 seconds. The pneumatic vibrator is activated on the packaging article at the same time to separate the powder from the walls and shaking is continued for 30 seconds, which is about 80 pulses.

| | Formula according to the invention | Comparative formula |
|---|---|---|
| Water | 57.910 | 57.910 |
| Glycerin | 5.600 | 5.600 |
| culture metalites | 0.008 | 0.008 |
| Colorants | 0.001 | 0.001 |
| Preservatives | Qs | Qs |
| petrolatum oil | 6.600 | 6.600 |
| almond oil | 0.008 | 0.008 |
| cetylstearylic alcohol | 3.200 | 3.200 |
| avocado oil | 0.008 | 0.008 |
| jojoba oil | 0.008 | 0.008 |
| shea butter | 0.480 | 0.480 |
| apricot oil | 0.008 | 0.008 |
| paraffin oil | 3.600 | 3.600 |
| coconut oil | 0.008 | 0.008 |
| carboxyvinyl polymer | 0.240 | 0.240 |
| polydimethysiloxane | 0.240 | 0.240 |
| fatty acids | 0.400 | 0.400 |
| glyceryl stearate and PEG-100 stearate | 0.800 | 0.800 |
| vitamin E | 0.080 | 0.080 |
| sodium hydroxide | 0.080 | 0.080 |
| | | |
| Alumina (ALUMINA AS-EM marketed by SENSIENT) | 19.740 | 19.740 |
| silica aerogel (DOW CORNING VM-2270 AEROGEL FINE PARTICLES marketed by DOW CORNING) | 0.260 | - |
| Hydrophobic fumed silica (Aerosil R972) | - | 0.260 |
| **Total** | **100.000** | **100.000** |
| Aspect | Dense powder | Formation of a paste. A coarser powder is obtained |

The oily phase is present in each formula in a content of at least 14% by weight in relation to the total weight of the formula.

The formula according to the invention, i.e. comprising silica aerogel particles, forms a powder.

On the contrary, the comparative formula containing hydrophobic fumed silica, forms a paste and not a powder.

The powder fabricated with the formula according to the invention is spread in a dish, drying is done at ambient temperature for 24h.

The dried formulas are stable at ambient temperature.

The composition of the formula according to the invention over time is as follows:

| | Formula according to the invention (cream in powder form) | Formula according to the invention after 9h drying | Formula according to the invention after 24h drying | Formula according to the invention after 4d drying |
|---|---|---|---|---|
| **Cream** | | | | |
| Water | 57.910 | 42.857 | 23.909 | 7.338 |
| Glycerin | 5.600 | 7.619 | 10.124 | 12.329 |
| culture metalites | 0.008 | 0.011 | 0.014 | 0.018 |
| Colorants | 0.001 | 0.002 | 0.002 | 0.003 |
| Preservatives | Qs | Qs | Qs | Qs |
| petrolatum oil | 6.600 | 8.952 | 11.932 | 14.530 |
| almond oil | 0.008 | 0.011 | 0.014 | 0.018 |
| cetylstearylic alcohol | 3.200 | 4.343 | 5.785 | 7.045 |
| avocado oil | 0.008 | 0.011 | 0.014 | 0.018 |
| jojoba oil | 0.008 | 0.011 | 0.014 | 0.018 |
| shea butter | 0.480 | 0.651 | 0.868 | 1.057 |
| apricot oil | 0.008 | 0.011 | 0.014 | 0.018 |
| paraffin oil | 3.600 | 4.886 | 6.508 | 7.926 |
| coconut oil | 0.008 | 0.011 | 0.014 | 0.018 |
| carboxyvinyl polymer | 0.240 | 0.326 | 0.434 | 0.528 |
| polydimethysiloxane | 0.240 | 0.326 | 0.434 | 0.528 |
| fatty acids | 0.400 | 0.543 | 0.723 | 0.881 |
| glyceryl stearate and PEG-100 stearate | 0.800 | 1.086 | 1.446 | 1.761 |
| vitamin E | 0.080 | 0.110 | 0.145 | 0.176 |
| sodium hydroxide | 0.080 | 0.110 | 0.145 | 0.176 |
| | | | | |
| Alumina (ALUMINA AS-EM marketed by SENSIENT) | 19.740 | 26.691 | 35.660 | 43.297 |
| silica aerogel (DOW CORNING VM-2270 AEROGEL FINE PARTICLES marketed by DOW CORNING) | 0.260 | 0.452 | 0.497 | 0.734 |
| **Total** | **100.000** | **100.00** | **100.000** | **100.000** |
| Aspect | Dense powder | Slightly dense powder | Fine and uniform powder | Coarse and dense powder |

The texture (dried concentrated emulsion) obtained is transformed into a cream with water remaining on the hands.

## Claims

1. Cosmetic composition in powder form, in which each powder grain is composed of:
- a dispersion of oily phase droplets in an aqueous phase, the oily phase being present in a content of at least 12% by weight of the total weight of the composition, and comprising at least one mineral oil, and
- a coating, comprising at least some hydrophobic silica aerogel particles, wherein the coating also comprises nanoparticles containing alumina.

2. Composition according to claim 1, in which the content of the aqueous phase is between 10% and 75% by weight of the total weight of the composition, preferably between 15% and 70% by weight.

3. Composition according to any one of the previous claims, in which the content of the oily phase is between 13% and 40% by weight of the total weight of the composition.

4. Composition according to any one of the previous claims, in which the content of hydrophobic silica aerogel particles is between 0.01 and 5% by weight, preferably between 0.1 and 3% by weight, and even better between 0.2 and 1% by weight in relation to the total weight of the composition.

5. Composition according to any one of claims 1 to 4, in which the content of nanoparticles containing alumina is between 15% and 60% by weight, preferably between 17% and 50% by weight, and even better between 18 and 45% by weight in relation to the total weight of the composition.

6. Composition according to any one of claims 1 to 5, in which the weight ratio (hydrophobic silica aerogel particles): (nanoparticles containing alumina) is between 0.005 and 0.03, and preferably between 0.01 and 0.02.

7. Composition according to any one of the previous claims, in which the oily phase comprises at least one non-volatile mineral oil and at least one non-volatile plant hydrocarbon oil.

8. Composition according to any one of the previous claims, in which the non-volatile plant hydrocarbon oil is chosen from among glyceride triesters, and particularly triesters of fatty acids and glycerol with fatty acids having linear or branched, saturated or unsaturated chain lengths ranging from 4 to 24 carbon atoms.

9. Composition according to claim 7 or 8, in which the non-volatile plant hydrocarbon oil is chosen from among wheat germ, sunflower, grape seed, sesame, corn, apricot, castor, shea, avocado, olive, soybean oils, almond and particularly sweet almond, palm, rapeseed, cotton, hazelnut, macadamia, jojoba, alfalfa, poppy seed, pumpkin, sesame, squash, rapeseed, blackcurrant, evening primrose, millet, barley, quinoa, rye, safflower, candlenut, passiflora, musk rose, coconut oils; or caprylic/capric acid triglycerides.

10. Composition according to any one of claims 7 to 9, in which the oily phase also comprises at least one lipophilic compound chosen from among fatty acids solid at ambient temperature with a linear carbon chain with 12 to 26 carbon atoms, silicone oils, fatty acids comprising 8 to 30 carbon atoms, waxes, pasty compounds, and mixtures thereof.

11. Composition according to any one of the previous claims, comprising the following by weight in relation to the total weight of the composition:
- from 55% to 70% of aqueous phase;
- from 12% to 20% by weight of oily phase; and,
- from 15% to 25% by weight of coating comprising hydrophobic silica aerogel particles and nanoparticles containing alumina.

12. Composition according to any one of claims 1 to 10, comprising the following by weight in relation to the total weight of the composition:
- from 15% to 40% of aqueous phase;
- from 20% to 35% by weight of oily phase; and,
- from 30% to 50% by weight of coating comprising hydrophobic silica aerogel particles and nanoparticles containing alumina.

13. Composition according to any one of claims 1 to 10, comprising the following by weight in relation to the total weight of the composition:
- from 30% to 60% of aqueous phase;
- from 15% to 25% by weight of oily phase; and,
- from 20% to 40% by weight of coating comprising hydrophobic silica aerogel particles and nanoparticles containing alumina.

14. Method of preparing a composition according to any one of the previous claims, comprising the mixture of the oily phase, the aqueous phase and the coating, and optionally drying of the mixture obtained.

## Patentansprüche

1. Kosmetische Zusammensetzung in Pulverform, bei der jedes Pulverkorn aus folgendem besteht:
- einer Dispersion aus Tröpfchen öliger Phase in einer wässrigen Phase, wobei die ölige Phase in einem Anteil von mindestens 12 Gewichtsprozent des Gesamtgewichts der Zusammensetzung vorhanden ist und mindestens ein Mineralöl umfasst, und
- einer Beschichtung, umfassend mindestens einige hydrophobe Silica-Aerogel-Partikel umfasst, wobei die Beschichtung auch Nanopartikel umfasst, die Aluminiumoxid enthalten.

2. Zusammensetzung nach Anspruch 1, wobei der Gehalt der wässrigen Phase zwischen 10 und 75 Gewichtsprozent des Gesamtgewichts der Zusammensetzung, vorzugsweise zwischen 15 und 70 Gewichtsprozent, ist.

3. Zusammensetzung nach einem der vorherigen Ansprüche, wobei der Gehalt der Ölphase zwischen 13 und 40 Gewichtsprozent des Gesamtgewichts der Zusammensetzung ist.

4. Zusammensetzung nach einem der vorherigen Ansprüche, in der der Gehalt an hydrophoben Silica-Aerogel-Partikel zwischen 0,01 und 5 Gewichtsprozent, vorzugsweise zwischen 0,1 und 3 Gewichtsprozent und noch besser zwischen 0,2 und 1 Gewichtsprozent, bezogen auf das Gesamtgewicht der Zusammensetzung, ist.

5. Zusammensetzung nach einem der Ansprüche 1 bis 4, wobei der Gehalt an Aluminiumoxid enthaltenden Nanopartikeln zwischen 15 und 60 Gewichtsprozent, vorzugsweise zwischen 17 und 50 Gewichtsprozent und noch besser zwischen 18 und 45 Gewichtsprozent, bezogen auf das Gesamtgewicht der Zusammensetzung, ist.

6. Zusammensetzung nach einem der Ansprüche 1 bis 5, wobei das Gewichtsverhältnis (hydrophobe Silica-Aerogel-Partikel): (Aluminiumoxid enthaltende Nanopartikel) zwischen 0,005 und 0,03, vorzugsweise zwischen 0,01 und 0,02, ist.

7. Zusammensetzung nach einem der vorherigen Ansprüche, wobei die Ölphase mindestens ein nicht flüchtiges Mineralöl und mindestens ein nicht flüchtiges pflanzliches Kohlenwasserstofföl umfasst.

8. Zusammensetzung nach einem der vorherigen Ansprüche, wobei das nichtflüchtige pflanzliche Kohlenwasserstofföl ausgewählt ist aus Glyceridtriestern und insbesondere Triestern von Fettsäuren und Glycerin, wobei die Fettsäuren lineare oder verzweigte, gesättigte oder ungesättigte Kettenlängen von 4 bis 24 Kohlenstoffatomen aufweisen.

9. Zusammensetzung nach Anspruch 7 oder 8, wobei das nichtflüchtige pflanzliche Kohlenwasserstofföl ausgewählt ist aus Weizenkeim-, Sonnenblumen-, Traubenkern-, Sesam-, Mais-, Aprikosen-, Rizinus-, Shea-, Avocado-, Oliven-, Sojaöl, Mandel- und insbesondere Süßmandel-, Palm-, Raps-, Baumwoll-, Haselnuss-, Macadamia-, Jojoba-, Alfalfa-, Mohn-, Kürbis-, Sesam-, Kürbis-, Raps-, Johannisbeere-, Nachtkerzen-, Hirse-, Gersten-, Quinoa-, Roggen-, Distel-, Kerzen-, Passiflora-, Moschusrosen-, Kokosnussöl; oder Caprylic/Caprinsäure-Triglyceriden.

10. Zusammensetzung nach einem der Ansprüche 7 bis 9, wobei die Ölphase auch mindestens eine lipophile Verbindung umfasst, die ausgewählt ist aus bei Raumtemperatur festen Fettsäuren mit einer linearen Kohlenstoffkette mit 12 bis 26 Kohlenstoffatomen, Silikonölen, Fettsäuren mit 8 bis 30 Kohlenstoffatomen, Wachsen, pastösen Verbindungen und deren Gemischen.

11. Zusammensetzung nach einem der vorherigen Ansprüche, umfassend Folgendes in Gewichtsanteilen bezogen auf das Gesamtgewicht der Zusammensetzung:
- von 55 % bis 70 % wässrige Phase;
- von 12 bis 20 Gewichtsprozent ölige Phase; und,
- von 15 bis 25 Gewichtsprozent Beschichtung, umfassend hydrophobe Silica-Aerogel-Partikel und aluminiumoxidhaltige Nanopartikel.

12. Zusammensetzung nach einem der Ansprüche 1 bis 10, umfassend Folgendes in Gewichtsanteilen bezogen auf das Gesamtgewicht der Zusammensetzung:
- von 15 % bis 40 % wässrige Phase;
- von 20 bis 35 Gewichtsprozent ölige Phase; und,
- von 30 bis 50 Gewichtsprozent Beschichtung, umfassend hydrophobe Silica-Aerogel-Partikel und aluminiumoxidhaltige Nanopartikel.

13. Zusammensetzung nach einem der Ansprüche 1 bis 10, umfassend Folgendes in Gewichtsanteilen bezogen auf das Gesamtgewicht der Zusammensetzung:
- von 30 % bis 60 % wässrige Phase;
- von 15 bis 25 Gewichtsprozent ölige Phase; und,
- von 20 bis 40 Gewichtsprozent Beschichtung, umfassend hydrophobe Silica-Aerogel-Partikel und aluminiumoxidhaltige Nanopartikel.

14. Verfahren zur Herstellung einer Zusammensetzung nach einem der vorherigen Ansprüche, umfassend das Mischen der öligen Phase, der wässrigen Phase und der Beschichtung und optional Trocknen des erlangten Gemischs.

## Revendications

1. Composition cosmétique sous forme de poudre, dans laquelle chaque grain de poudre est constitué par :
- une dispersion de gouttelettes de phase huileuse dans une phase aqueuse, la phase huileuse étant présente en une teneur d'au moins 12% en poids par rapport au poids total de composition, et comprenant au moins une huile minérale, et
- un enrobage comprenant au moins des particules d'aérogel de silice hydrophobe, dans laquelle l'enrobage comprend en outre des nanoparticules contenant de l'alumine.

2. Composition selon la revendication 1, dans laquelle la teneur en phase aqueuse est comprise entre 10% et 75% en poids par rapport au poids total de composition, de préférence entre 15% et 70% en poids.

3. Composition selon l'une des revendications précédentes, dans laquelle la teneur en phase huileuse est comprise entre 13% et 40% en poids par rapport au poids total de composition.

4. Composition selon l'une des revendications précédentes, dans laquelle la teneur en particules d'aérogel de silice hydrophobe est comprise entre 0,01 et 5% en poids, de préférence entre 0,1 et 3% en poids, préférentiellement entre 0,2 et 1% en poids par rapport au poids total de la composition.

5. Composition selon l'une des revendications 1 à 4, dans laquelle la teneur en nanoparticules contenant de l'alumine est comprise entre 15% et 60% en poids, et de préférence entre 17% et 50% en poids, préférentiellement entre 18 et 45% en poids par rapport au poids total de la composition.

6. Composition selon l'une des revendications 1 à 5, dans laquelle le ratio pondéral (particules d'aérogel de silice hydrophobe) : (nanoparticules contenant de l'alumine) est compris entre 0,005 et 0,03, et de préférence entre 0,01 et 0,02.

7. Composition selon l'une des revendications précédentes, dans laquelle la phase huileuse comprend au moins une huile minérale non volatile et au moins une huile hydrocarbonée d'origine végétale non volatile.

8. Composition selon la revendication précédente, dans laquelle l'huile hydrocarbonée d'origine végétale non volatile est choisie parmi les triesters de glycérides, en particulier les triesters d'acides gras et de glycérol dont les acides gras ont des longueurs de chaînes, linéaires ou ramifiées, saturées ou insaturées, allant de 4 à 24 atomes de carbone.

9. Composition selon la revendication 7 ou 8, dans laquelle l'huile hydrocarbonée d'origine végétale non volatile est choisie parmi les huiles de germe de blé, de tournesol, de pépins de raisin, de sésame, de maïs, d'abricot, de ricin, de karité, d'avocat, d'olive, de soja, l'huile d'amande et notamment l'huile d'amande douce, de palme, de colza, de coton, de noisette, de macadamia, de jojoba, de luzerne, de pavot, de potimarron, de sésame, de courge, de colza, de cassis, d'onagre, de millet, d'orge, de quinoa, de seigle, de carthame, de bancoulier, de passiflore, de rosier muscat, de noix de coco ; et les triglycérides des acides caprylique/caprique.

10. Composition selon l'une des revendications 7 à 9, dans laquelle la phase huileuse comprend en outre au moins un composé lipophile choisi parmi les alcools gras solides à température ambiante à chaîne carbonée linéaire ayant de 12 à 26 atomes de carbone, les huiles siliconées, les acides gras comportant de 8 à 30 atomes de carbone, les cires, les composés pâteux et leurs mélanges.

11. Composition selon l'une des revendications précédentes, comprenant, en poids par rapport au poids total de la composition :
- de 55% à 70% de phase aqueuse ;
- de 12% à 20% en poids de phase huileuse ; et
- de 15% à 25% en poids d'enrobage comprenant des particules d'aérogel de silice hydrophobe et des nanoparticules contenant de l'alumine.

12. Composition selon l'une des revendications 1 à 10, comprenant, en poids par rapport au poids total de la composition :
- de 15% à 40% de phase aqueuse ;
- de 20% à 35% en poids de phase huileuse ; et
- de 30% à 50% en poids d'enrobage comprenant des particules d'aérogel de silice hydrophobe et des nanoparticules contenant de l'alumine.

13. Composition selon l'une des revendications 1 à 10, comprenant, en poids par rapport au poids total de la composition :
- de 30% à 60% de phase aqueuse ;
- de 15% à 25% en poids de phase huileuse ; et
- de 20% à 40% en poids d'enrobage comprenant des particules d'aérogel de silice hydrophobe et des nanoparticules contenant de l'alumine.

14. Procédé de préparation d'une composition selon l'une des revendications précédentes, comprenant le mélange de la phase huileuse, de la phase aqueuse et de l'enrobage, et optionnellement le séchage du mélange obtenu.
